# EUROPEAN PATENT SPECIFICATION

(11) **EP 2 688 638 B1**
(45) Date of publication and mention of the grant of the patent: **21.01.2015**
(21) Application number: 12713456.7
(22) Date of filing: 16.03.2012
(51) Int. Cl.: A61N 1/05

(54) **IMPLANTABLE AUDITORY PROSTHESIS WITH TEMPORARY CONNECTOR**
IMPLANTIERBARE HÖRPROTHESE MIT TEMPORÄREM VERBINDER
PROTHÈSE AUDITIVE IMPLANTABLE AVEC CONNECTEUR TEMPORAIRE

(30) Priority: 25.03.2011 US 201161467472 P
(43) Date of publication of application: 29.01.2014
(73) Proprietor: Med-El Elektromedizinische Geraete GmbH, 6020 Innsbruck (AT)
(72) Inventor: JOLLY, Claude, 6020 Innsbruck (AT); LENARZ, Thomas, 30559 Hannover (DE)
(74) Representative: Lucke, Andreas
(86) International application number: PCT/US2012/029360
(87) International publication number: WO 2012/134834

(56) References cited:
- EP-A1- 2 113 283
- WO-A1-2010/151768

## Description

The present invention relates to medical implants, and more specifically to ear implant systems such as cochlear implants, auditory brainstem implants, and vestibular implants.

### BACKGROUND ART

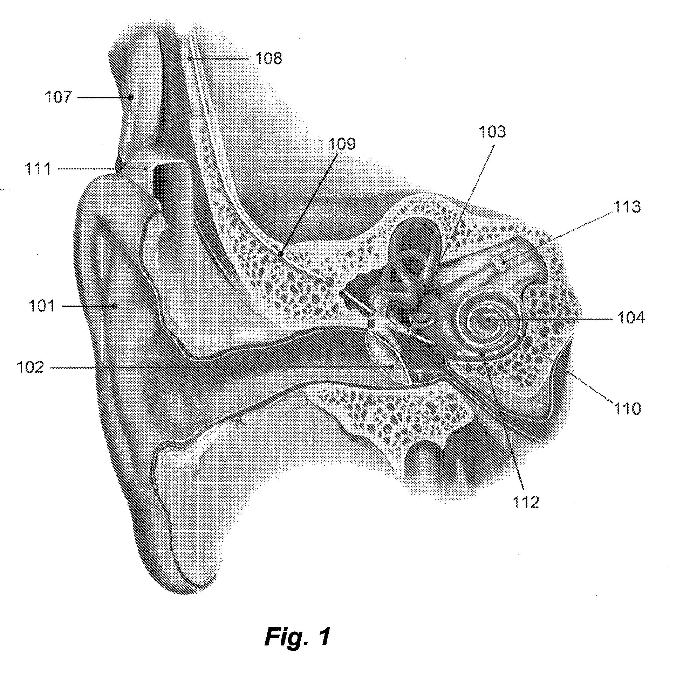

A normal ear transmits sounds as shown in Figure 1 through the outer ear **101** to the tympanic membrane (eardrum) **102,** which moves the bones of the middle ear **103,** which in turn vibrate the oval window and round window openings of the cochlea **104.** The cochlea **104** is a long narrow duct wound spirally about its axis for approximately two and a half turns. The cochlea **104** includes an upper channel known as the scala vestibuli and a lower channel known as the scala tympani, which are connected by the cochlear duct. The scala tympani forms an upright spiraling cone with a center called the modiolar where the spiral ganglion cells of the acoustic nerve **113** reside. In response to received sounds transmitted by the middle ear **103,** the fluid filled cochlea **104** functions as a transducer to generate electric pulses that are transmitted to the cochlear nerve **113,** and ultimately to the brain. Hearing is impaired when there are problems in the ability to transduce external sounds into meaningful action potentials along the neural substrate of the cochlea **104.**

In some cases, hearing impairment can be addressed by an auditory prosthesis system such as a cochlear implant that electrically stimulates auditory nerve tissue with small currents delivered by multiple electrode contacts distributed along an implant electrode. Figure 1 shows some components of a typical cochlear implant system where an external microphone provides an audio signal input to an external signal processing stage 111 which implements one of various known signal processing schemes. The processed signal is converted by the external signal processing stage **111** into a digital data format, such as a sequence of data frames, for transmission into a receiver processor in an implant housing **108.** Besides extracting the audio information, the receiver processor in the implant housing **108** may perform additional signal processing such as error correction, pulse formation, etc., and produces a stimulation pattern (based on the extracted audio information) that is sent through an electrode lead **109** to an implanted electrode array **112** which penetrates into the cochlea **104** through a surgical opening called a cochleostomy. Typically, this electrode array **112** includes multiple electrode contacts **110** on its surface that deliver the stimulation signals to adjacent neural tissue of the cochlea **104** which the brain of the patient interprets as sound. The individual electrode contacts **110** may be activated sequentially, or simultaneously in one or more contact groups.

In some patients, the cochlea **104** itself is damaged so that a cochlear implant system as such is not a treatment option. One alternative for some such patients is an Auditory Brain Stem Implant (ABI) system which is somewhat like a cochlear implant, but which instead implants a stimulation electrode into auditory tissue in the brainstem of the patient.

Insertion and placement and insertion of the electrode array **112** into the cochlea **104** (or the auditory brainstem tissue in the case of an ABI electrode) causes trauma to the target tissue due to the rigidity, friction, and impact of moving the device through the cochlea **104.** For example, insertion of the electrode array **112** may damage soft tissues, membranes, thin bony shelves, blood vessels, neural elements, etc. In the case of multiple insertions, the damage can accumulate. Thus, designers of the electrode array **110** work hard to ensure that it is soft and flexible to minimize the insertion trauma.

WO 2010/151768 A1 discloses an insertion system for inserting an implantable electrode carrier. The system includes an insertion instrument having a housing and a vibration generator coupled adjacent to the housing. The instrument comprises one or more sensors positioned near the end of the instrument housing where the instrument is held by the surgeon. In one embodiment, the sensor can be a force sensor sensing a force applied to the instrument, and the vibration generator is configured to control vibration paramenters based on the sensed force.

### SUMMARY

Embodiments of the present invention are directed to an implantable electrode arrangement for an ear implant system. A proximal electrode lead has electrode wires for carrying one or more electrical stimulation signals. A distal electrode array has one or more electrode contacts each forming a terminal end of an electrode wire for applying the electrical stimulation signals to target neural tissue, and one or more intra-operative sensors for generating insertion sensing signals during surgical insertion or placing of the electrode array into or onto the target tissue. An intra-operative electrode section has a sensor connector for providing a temporary connection of one or more external measurement arrangements to the one or more intra-operative sensors during the surgical insertion of the electrode array without being functional after the surgical insertion.

In further specific embodiments, the insertion signals include dynamic real time sensing signals. The intra-operative sensors may include optical sensors, inductive sensors, and chemical sensors, and at least one of the intra-operative sensors may be separate from the electrode contacts. There also may be a connector cap for covering the sensor connector following the surgical insertion of the electrode array into the target tissue. The connector cap may be adapted to electrically isolate and/or electrically connect any terminal wire ends enclosed within.

The ear implant system may include a cochlear implant system, an auditory brainstem implant system, or a vestibular implant system. And embodiments of the present invention also include an ear implant system having an electrode arrangement according to any of the above.

These objects of the invention are solved by the subject matter as claimed by the independents claims 1 and 14. Various embodiments of the invention are subject of the dependent claims.

### BRIEF DESCRIPTION OF THE DRAWINGS

Figure 1 shows various anatomical structures in a human ear and some components of a typical cochlear implant system.
Figure 2 shows an example of one specific embodiment of the present invention.
Figure 3 shows details of an ABI electrode array according to one embodiment.
Figure 4 shows details of a connector cap according to one embodiment.
Figure 5 shows details of a cochlear implant electrode array according to one embodiment.

### DETAILED DESCRIPTION

Design engineers try to design the most atraumatic implant electrodes and insertion techniques so that new patients with residual hearing can have access to auditory prosthesis implants without degradation of their existing hearing. But many measurements cannot be performed from the implant itself except for potential field measurements which are a crude measurement dedicated to the whole nerve action potential and retransmission by telemetry which is not a real time measurement. More specifically, conventional telemetry measurements with the implant device are limited to extra cellular potential field generated by intra cellular action potential, or to recording potential distribution caused by current generated at one or more electrodes and recorded with the other electrodes. All signals are processed somewhat crudely by the implant electronics which does not allow for more precise or sophisticated measurements. Only a static measurement is available and no intra-operative real time measurement is possible as electrode insertion proceeds.

It would be most useful to provide an auditory prosthesis implant which could perform sophisticated dynamic measurements during implantation of the electrode in the target tissue. For example, during insertion of a cochlear implant electrode, such measurements could reveal forces, potential changes in the cochlear structure during insertion and placement of the electrode, allow recording of cochlear microphonics, chemical changes, concentration of some molecular species, pH changes etc. Having intra-operative access to such measurements would allow evaluation of the quality of the electrode insertion, preservation of tissue, amount of bleeding and injury inflicted on the tissue, perforation of basilar membrane and mixing of perilymph and endolymph, forces applied at different location, molecular generation of sub species, bleeding etc. These measurements would allow better understanding of the mechanism of insertion trauma with electrodes in real time.

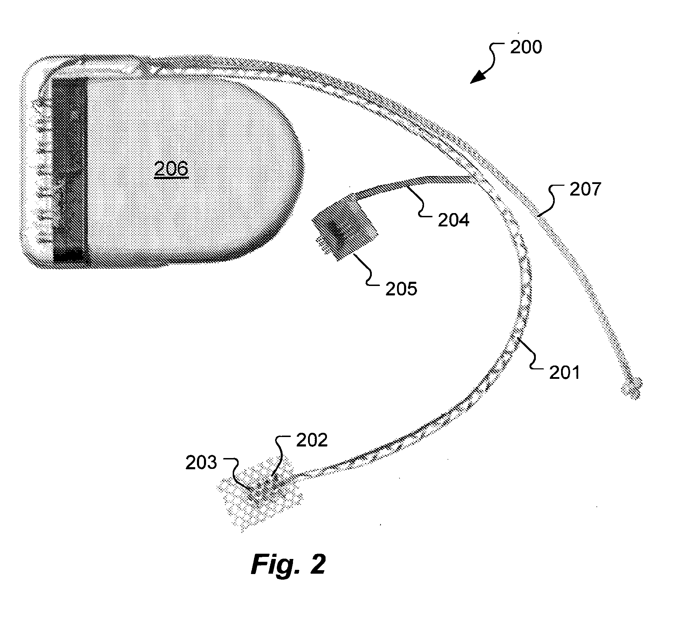

Embodiments of the present invention are directed to an improved implantable electrode arrangement for an auditory prosthesis system such as a cochlear implant or ABI system which allows for taking real-time intra-operative measurements as the implant electrode is inserted into or placed into or onto the target tissue. Figure 2 shows an example of one specific embodiment of an ABI implant stimulator **206** which produces one or more electrical stimulation signals that are carried by electrode wires in an electrode lead **201** which connected at its base to the housing of the implant stimulator **206.** At the apical end of the electrode lead **201** is an electrode array **202** with two types of contact surfaces **203.** There is also a ground electrode that may be either part of the implant stimulator **206** or a separate ground electrode **207.**

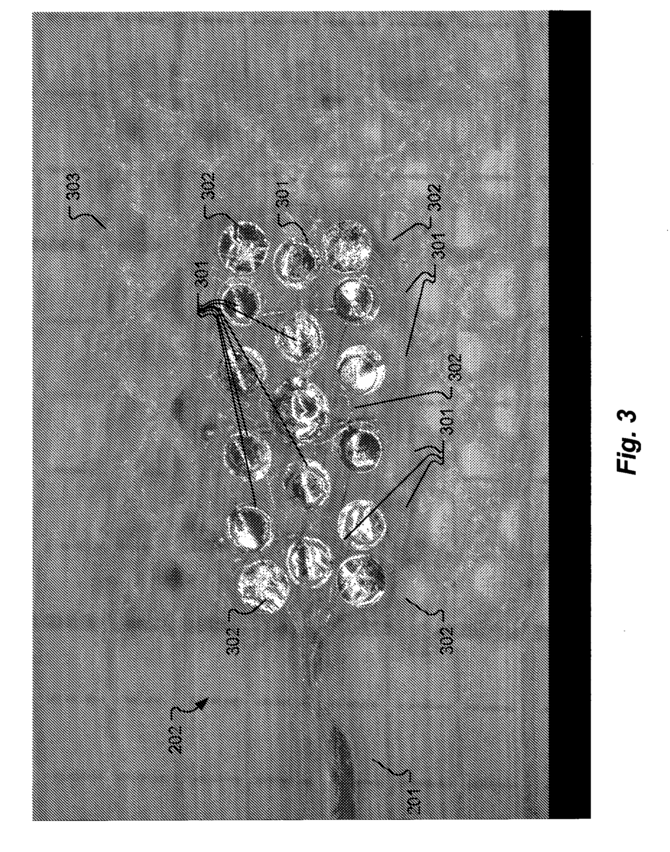

Figure 3 shows the distal electrode array **202** in greater detail for an ABI implant system where there are conventional electrode contacts **301** that form a terminal end of each electrode wire for applying the electrical stimulation signals to the target neural tissue. Intra-operative sensors **302** can be at any specific position within the electrode array **202** and generate intra-operative sensing signals during surgical insertion of the electrode array **202** into the target tissue. In one specific embodiment, the intra-operative sensors **302** are on the edges of the electrode array **202.** In a further embodiment, a further sensor **302** is in or close to the center of the electrode array **202.** For example, the intra-operative sensors **302** could be separate electrodes for recording potential changes resulting from any loss of endocochlear potential (100 mV positive potential in the endolymph compared to perilymph). Or the intra-operative sensors **302** may specifically include a chemical or pH sensor. Or the intra-operative sensors **302** may be inductive sensors based on wire coils in the electrode array **202** and/or electrode lead **201** that sense changes in a magnetic field induced in near the target tissue. Besides electrical sensing contacts, the intra-operative sensors **302** also could be optical sensors connected to optical fibers. Figure 3 also shows an array backing **303** of mesh fabric that provides a structure for post-operative tissue growth that helps permanently secure the electrode array **202** in position.

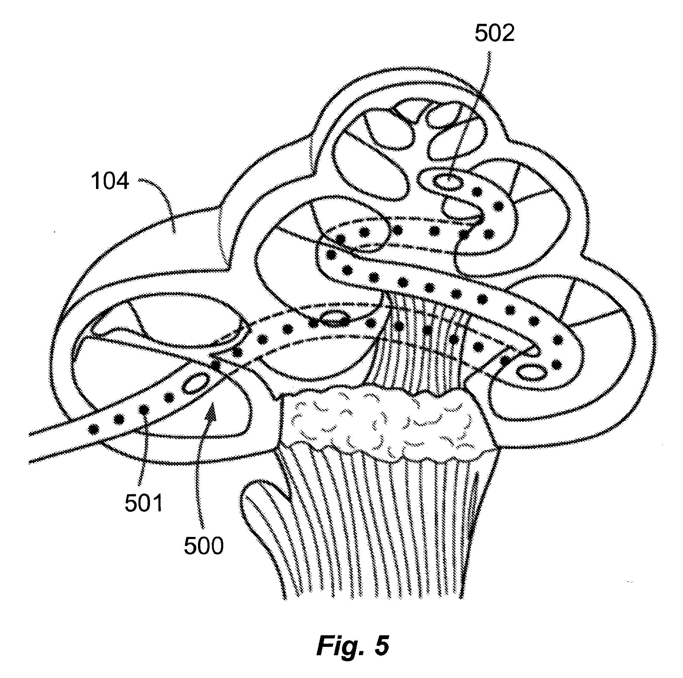

Figure 5 shows the intracochlear electrode array 500 of a cochlear implant system, having conventional electrode contacts **501** that form a terminal end of each electrode wire for applying the electrical stimulation signals to the target neural tissue and intra-operative sensors **502.** In a preferred embodiment the intra operative sensors **502** are equally spaced apart over the electrode array **500.** The intra-operative sensors may be electrode contacts, preferred the same as the conventional electrode contacts **501,** or fiber-optic terminal ends. In a further embodiment, the intra-operative sensors **502** are only on a part of the electrode array **500,** e.g. the distal end of the electrode array **500.**

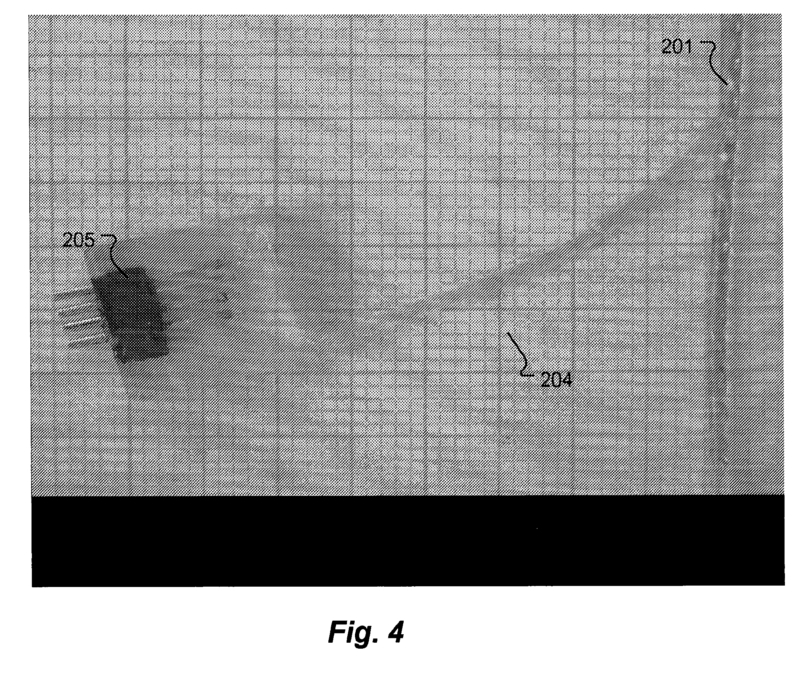

The electrode lead **201** also has a separate intra-operative electrode branch **204** that is connected to the intra-operative sensors **302.** Intra-operative electrode branch **204** terminates in a sensor connector **205,** in this case a 4 pin AXON connector for temporary connection of one or more external measurement arrangements during the surgical insertion of the electrode array **202.** Figure 4 shows the intra-operative electrode branch **204** and the sensor connector **205** in greater detail. The intra-operative electrode branch **204** and the sensor connector **205** are connected through another cable to sophisticated recording equipment located in the operating room for the intra-operative measurements. At the end of the surgery, the intra-operative electrode branch **204** is no longer needed and can be cut where it joins the electrode lead **201.** Alternatively, the sensor connector **205** can be simply left in place and covered by a biocompatible cap made of silicone or other biocompatible material to protect it from surrounding body fluids. Such a cap may short circuit the connector pins or isolates them to each other. The sensor connector **205** pins may be of platinum-iridium alloy or other biocompatible material. In this way the covered sensor connector **205** could be placed in the mastoid cavity used again later in a subsequent surgery as needed.

Although various exemplary embodiments of the invention have been disclosed, it should be apparent to those skilled in the art that various changes and modifications can be made which will achieve some of the advantages of the invention without departing from the true scope of the invention.

## Claims

1. An implantable electrode arrangement for an ear implant system having a proximal and a distal end, the distal end being the leading end upon insertion, said implantable electrode arrangement comprising:
a proximal electrode lead (201) having a plurality of electrode wires for carrying one or
more electrical stimulation signals;
a distal electrode array (202) having:
i. one or more electrode contacts (301) each forming a terminal end of an electrode wire for applying the electrical stimulation signals to target neural tissue, and **characterized in that** said electrode array further comprises
ii. one or more intra-operative sensors (302) for generating insertion sensing signals during surgical insertion or placing of the electrode array (202) into or onto the target tissue; and said electrode arrangement further comprises an intra-operative section (204) having a sensor connector (205) for providing a temporary
connection of one or more external measurement arrangements to the one or more intra-operative sensors (302) during the surgical insertion of the electrode array (202) without being functional after the surgical insertion.

2. An electrode arrangement according to claim 1, wherein at least one of the intra-operative sensors (302) is separate from the electrode contacts (301).

3. An electrode arrangement according to claim 1 or 2, wherein the insertion signals include dynamic real time sensing signals.

4. An electrode arrangement according to any of claims 1 to 3, wherein the intra-operative section (204) is an electrode section.

5. An electrode arrangement according to any of claims 1 to 3, wherein the intra-operative section (204) is an optical section.

6. An electrode arrangement according to claim 5, wherein at least one of the intra-operative sensors (302) is an optical sensor.

7. An electrode arrangement according to claim 4, wherein at least one of the intra-operative sensors (302) is an inductive sensor.

8. An electrode arrangement according to claim 4, wherein at least one of the intra-operative sensors (302) is a chemical sensor.

9. An electrode arrangement according to claim 1, further comprising:
a connector cap for covering the sensor connector (205) following the surgical insertion of the electrode array (202) into the target tissue.

10. An electrode arrangement according to claim 9, wherein the connector cap further is adapted to electrically isolate any terminal wire ends enclosed within.

11. An electrode arrangement according to claim 9, wherein the connector cap further is adapted to electrically connect together any terminal wire ends enclosed within.

12. An electrode arrangement according to any of claims 1 to 11, wherein the ear implant system includes a cochlear implant system.

13. An electrode arrangement according to any claims 1 to 11, wherein the ear implant system includes an auditory brainstem implant system (206).

14. An electrode arrangement according to any claims 1 to 11, wherein the ear implant system includes a vestibular implant system.

15. An ear implant system having an electrode arrangement according to any of claims 1-14.

## Patentansprüche

1. Implantierbare Elektrodenanordnung für ein Ohrimplantatsystem mit einem proximalen und einem distalen Ende, wobei das distale Ende das beim Einführen führende Ende ist, wobei die implantierbare Elektrodenanordnung Folgendes umfasst:
eine proximale Elektrodenleitung (201) mit einer Mehrzahl von Elektrodendrähten zum Übertragen von einem oder mehreren elektrischen Stimulationssignalen;
ein distales Elektronen-Array (202), umfassend:
i. einen oder mehrere Elektrodenkontakte (301), die jeweils eine Endstelle eines Elektrodendrahtes bilden, um die elektronischen Stimulationssignale an ein neurales Zielgewebe anzulegen, und **dadurch gekennzeichnet, dass** das Elektroden-Array ferner Folgendes umfasst:
ii. eine oder mehrere intraoperative Sensoren (302) zum Erzeugen von Einführungs-Fühlsignalen während der chirurgischen Einführung oder dem Anordnen des Elektroden-Arrays (202) in oder auf dem Zielgewebe; und dass die Elektrodenanordnung ferner Folgendes umfasst:
einen intraoperativen Abschnitt (204) mit einem Sensor-Verbinder (205) zum Bereitstellen einer vorübergehenden Verbindung von einer oder mehreren externen Messanordnungen mit dem einen oder mehreren intraoperativen Sensoren (302) während des chirurgischen Einführens des Elektroden-Arrays (202), der nach dem chirurgischen Einführen keine Funktion mehr hat.

2. Elektrodenanordnung nach Anspruch 1, bei der zumindest einer der intraoperativen Sensoren (302) von den Elektrodenkontakten (301) getrennt ist.

3. Elektrodenanordnung nach Anspruch 1 oder 2, bei der die Einführsignale dynamische Echtzeitfühlsignale umfassen.

4. Elektrodenanordnung nach einem der Ansprüche 1 bis 3, bei der der intraoperative Abschnitt (204) ein Elektrodenabschnitt ist.

5. Elektrodenanordnung nach einem der Ansprüche 1 bis 3, bei dem der intraoperative Abschnitt (204) ein optischer Abschnitt ist.

6. Elektrodenanordnung nach Anspruch 5, bei der zumindest einer der intraoperativen Sensoren (302) ein optischer Sensor ist.

7. Elektrodenanordnung nach Anspruch 4, bei der zumindest einer der intraoperativen Sensoren (302) ein induktiver Sensor ist.

8. Elektrodenanordnung nach Anspruch 4, bei der zumindest einer der intraoperativen Sensoren (302) ein chemischer Sensor ist.

9. Elektrodenanordnung nach Anspruch 1, die ferner Folgendes umfasst:
eine Verbinderkappe zum Abdecken des Sensorvermittlers (205) nach der chirurgischen Einführung des Elektroden-Arrays (202) in das Zielgewebe.

10. Elektrodenanordnung nach Anspruch 9, bei der die Verbinderkappe ferner geeignet ist, Drahtendstellen zu isolieren.

11. Elektrodenanordnung nach Anspruch 9, bei der die Verbinderkappe ferner geeignet ist, sämtliche darin enthaltenen Drahtendstellen elektrisch zu verbinden.

12. Elektrodenanordnung nach einem der Ansprüche 1 bis 11, bei der das Ohrenimplantat ein Cochlearimplantatsystem umfasst.

13. Elektrodenanordnung nach einem der Ansprüche 1 bis 11, bei der das Ohrenimplantatsystem ein Hör-Hirnstammimplantatsystem (206) ist.

14. Elektrodenanordnung nach einem der Ansprüche 1 bis 11, bei der das Ohrimplantatsystem ein vestibulares Implantsystem umfasst.

15. Ohrimplantatsystem mit einer Elektrodenanordnung gemäß einem der Ansprüche 1 bis 14.

## Revendications

1. Agencement d'électrodes implantable pour un système d'implant auriculaire comportant une extrémité distale et une extrémité proximale, l'extrémité distale étant l'extrémité avant lors de l'insertion, ledit agencement d'électrodes implantable comprenant :
un conducteur d'électrode proximal (201) comportant une pluralité de fils d'électrode pour transporter un ou plusieurs signaux de stimulation électrique ;
un ensemble d'électrodes distales (202) comportant :
i. un ou plusieurs contacts d'électrode (301) formant chacun une extrémité terminale d'un fil d'électrode pour appliquer les signaux de stimulation électrique à un tissu neuronal cible, et **caractérisé en ce que** ledit ensemble d'électrodes comprend en outre :
ii. un ou plusieurs capteurs intraopératoires (302) pour générer des signaux de détection d'insertion pendant l'insertion chirurgicale ou le placement de l'ensemble d'électrodes (202) dans ou sur le tissu cible ; et ledit agencement d'électrode comprend en outre :
une section intraopératoire (204) comportant un connecteur de capteur (205) pour réaliser une connexion temporaire d'un ou de plusieurs agencements de mesure externes aux dits un ou plusieurs capteurs intraopératoires (302) pendant l'insertion chirurgicale de l'ensemble d'électrodes (202) sans être fonctionnelle après l'insertion chirurgicale.

2. Agencement d'électrodes selon la revendication 1, dans lequel au moins l'un des capteurs intraopératoires (302) est séparé des contacts d'électrode (301).

3. Agencement d'électrodes selon la revendication 1 ou 2, dans lequel les signaux d'insertion comprennent des signaux de détection dynamique en temps réel.

4. Agencement d'électrodes selon l'une quelconque des revendications 1 à 3, dans lequel la section intraopératoire (204) est une section d'électrodes.

5. Agencement d'électrodes selon l'une quelconque des revendications 1 à 3, dans lequel la section intraopératoire (204) est une section optique.

6. Agencement d'électrodes selon la revendication 5, dans lequel au moins l'un des capteurs intraopératoires (302) est un capteur optique.

7. Agencement d'électrodes selon la revendication 4, dans lequel au moins l'un des capteurs intraopératoires (302) est un capteur inductif.

8. Agencement d'électrodes selon la revendication 4, dans lequel au moins l'un des capteurs intraopératoires (302) est un capteur chimique.

9. Agencement d'électrodes selon la revendication 1, comprenant en outre :
un capuchon de connecteur pour recouvrir le connecteur de capteur (205) à la suite de l'insertion chirurgicale de l'ensemble d'électrodes (202) dans le tissu cible.

10. Agencement d'électrodes selon la revendication 9, dans lequel le capuchon de connecteur est en outre conçu pour isoler électriquement les extrémités terminales des fils enfermés dans celui-ci.

11. Agencement d'électrodes selon la revendication 9, dans lequel le capuchon de connecteur est en outre conçu pour connecter électriquement les unes aux autres les extrémités terminales des fils enfermés dans celui-ci.

12. Agencement d'électrodes selon l'une quelconque des revendications 1 à 11, dans lequel le système d'implant auriculaire comprend un système d'implant cochléaire.

13. Agencement d'électrodes selon l'une quelconque des revendications 1 à 11, dans lequel le système d'implant auriculaire comprend un système d'implant de tronc cérébral auditif (206).

14. Agencement d'électrodes selon l'une quelconque des revendications 1 à 11, dans lequel le système d'implant auriculaire comprend un système d'implant vestibulaire.

15. Système d'implant auriculaire comportant un agencement d'électrodes selon l'une quelconque des revendications 1 à 14.
